# EUROPEAN PATENT APPLICATION

(11) **EP 2 006 387 A1**
(43) Date of publication of application: **24.12.2008**
(21) Application number: 07012281.7
(22) Date of filing: 22.06.2007
(51) Int. Cl.: C12N 15/67, C12N 15/82, A01H 5/00

(54) **Translation control elements for plastid transformation**

(71) Applicant: Icon Genetics GmbH, 80333 München (DE)
(72) Inventor: Stettner, Cornelia, 84562 Mettenheim (DE); Herz, Stefan, 80636 München (DE); Golds, Timothy, 85354 Freising (DE)
(74) Representative: Blodig, Wolfgang

(57) **Abstract**

Nucleic acid comprising a translation control element and, downstream thereof, a non-yersinia phage coding sequence to be expressed in plant plastids or bacteria, said translation control element comprising
(i) a sequence of SEQ ID NO: 1;
(ii) a sequence of SEQ ID NO: 2; or
(iii) a sequence having a sequence identity of at least 75 % to the sequence of SEQ ID NO: 2;
or a complementary nucleic acid having a sequence complementary to that of said nucleic acid.

## Description

### FIELD OF INVENTION FIELD OF INVENTION

The present invention relates to a nucleic acid having a translation control element for expressing a sequence of interest in plant plastids or in bacteria. The invention further provides a vector for providing plants, plant tissue, plant cells or bacteria with said nucleic acid. Further provided are plants, plant tissue, or plant cells, and bacteria comprising said nucleic acid. Further provided are processes for expressing a coding sequence of interest in bacteria or in plastids of a plant, of plant tissue or of plant cells.

### BACKGROUND OF THE INVENTION

According to generally accepted knowledge, two classes of cell organelles, i.e. plastids and mitochondria, are derived from initially independent prokaryotes that were taken up into a predecessor of present day eukaryotic cells by separate endosymbiotic events (Gray, 1991). As a consequence, these organelles contain their own DNA, DNA transcripts in the form of messenger RNA, ribosomes, and at least some of the tRNAs that are required for decoding of genetic information (Marechal-Drouard et al., 1991).

Shortly after endosymbiotic uptake, these organelles were genetically autonomous since they contained all the elements necessary to drive prokaryotic life, however this autonomy was reduced during evolution by transfer of genetic information to the cell's nucleus. Nevertheless, their genetic information is of sufficient complexity to make cell organelles an attractive target for gene technology. This is particularly the case with plastids, since these organelles still encode about 50% of the proteins required for photosynthesis, their main function inside the plant cell. Plastids also encode their own ribosomal RNAs, the majority of their tRNAs and ribosomal proteins. In total, the number of genes in the plastome is in the range of 120 (Palmer, 1991). The vast majority of proteins that are found in plastids are, however, imported from the nuclear/cytosolic genetic compartment.

With the development of general molecular cloning technologies, it has become possible to genetically modify higher plants by transformation. The main emphasis in plant transformation was and still is on nuclear transformation, since the majority of genes, ca. 26.000 in the case of *Arabidopsis thaliana,* are found in the cell's nucleus. Nuclear transformation was easier to achieve, since biological vectors such as *Agrobacterium tumefaciens* were available, which could be modified efficiently to enable nuclear transformation (Galvin, 1998). In addition, the nucleus is more directly accessible to foreign nucleic acids, while the organelles are surrounded by two envelope membranes that are, generally speaking, not permeable to macromolecules such as DNA.

Technology for transforming plastids is highly desirable since it could make use of the high gene dosage in these organelles that offers the potential of extremely high expression levels of transgenes. In addition, plastid transformation is attractive because plastid-encoded traits are not pollen transmissible in most important crop species; hence, potential risks of inadvertent transgene escape to wild relatives of transgenic plants are largely reduced. Other potential advantages of plastid transformation include the feasibility of simultaneous expression of multiple genes as a polycistronic unit and the elimination of positional effects and gene silencing that may result following nuclear transformation.

Methods that allow stable transformation of plastids have been developed for higher plants. To date, two different methods are available, i.e. particle bombardment of tissues, in particular leaf tissues (Svab et al., 1990), and treatment of protoplasts with polyethylene glycol (PEG) in the presence of suitable transformation vectors (Golds et al., 1993). Both methods mediate the transfer of plasmid DNA across the two envelope membranes into the organelle's stroma.

Plastid transformation methods usually rely on transformation vectors in which one or more transgenes are flanked by plastome sequences directing the insertion of the foreign genes by homologous recombination. Expression of the introduced gene or genes is achieved by placing the coding region under the control of regulatory elements. These regulatory elements usually contain a promoter active in plastids operably linked to 5'- and 3'-untranslated regions (UTRs). The 5'-UTR contains sequence information for translation initiation (e.g. ribosome binding site) and is therefore also designated as a translation control element. The 3'-UTR acts mainly as a processing and stabilizing element (Stern & Gruissem, 1987) and is reported to be of only minor significance for controlling the expression level in transplastomic plants (Eibl et al., 1999). Promoters active in plastids can be obtained using plastome derived transcription activating sequences or using other sequences of synthetic or natural origin mediating transcription activity in the plastid. Examples for plastid promoters which mediate strong transcriptional activity are the rrn-promoter from the 16S-rRNA operon (Svab and Maliga, 1993) or the psbA promoter (Staub and Maliga, 1993). An example for a heterologous promoter is the phage T7 promoter which is, however, only active if the corresponding T7 RNA-polymerase is also present. Alternatively, it is possible to insert foreign coding region(s) into a transcriptionally active site of the plastome, thus expressing the introduced genes by operable linkage to promoters already present in the plastome (Staub & Maliga, 1995). It is then necessary to fuse the coding region to be expressed to a translation control element (Herz et al. 2005). In this case, the translation control element is located between adjacent genes which are under the control of the same promoter (polycistronic operon). Translation control elements in polycistronic operons are sometimes also referred to as spacer sequences.

Transcript levels of the introduced gene(s) depend on promoter activity and turnover rates of the mRNA. In the case where the introduced gene(s) are controlled by plastid derived promoter elements, transcription patterns resemble the transcription patterns of the corresponding plastid genes. If an artificial operon has been generated by introducing the transgene(s) into a transcriptionally active site of the plastome, transcription activity is determined by the corresponding upstream promoter.

Expression in plastids is predominantly controlled at the post-transcriptional level (Stern et al. 1997; Gruissem & Tonkyn, 1993). Therefore suitable translation control elements are important components of plastid expression vectors (Herz et al., 2005; Eibl et al., 1999). Most vectors contain intact or truncated 5'-UTRs of highly expressed plastid genes like *psbA, atpB* or *rbcL* (Staub & Maliga, 1993; Kuroda & Maliga, 2001 a). However, use of endogenous control elements for the expression of genes of interest in plastids carries the risk of undesired recombination events between the introduced and the original elements, because of the highly efficient recombination system in plastids (Svab & Maliga, 1993). Recombination between duplicated sequences can result in loop-out recombination or genome rearrangements depending on the relative orientation of the duplicated elements. An example of such a rearrangement was recently described for duplicated psbA-UTRs (Rogalski et al., 2006). To avoid this problem many research groups use control elements from related species e.g. rice sequences in tobacco (Reddy et al. 2002; Zhou et al., 2006). However, undesired recombination events cannot be totally excluded due to the intrinsic similarity between homologous elements from different species. Besides, a regulatory element from a different species is often inferior in terms of efficiency to the endogenous counterpart. The recombination problem can be circumvented with non-plastid elements, but very few translation control elements of non-plastid origin are known, have been tested and are used today (Table 1). All but one of the non-plastid translation control elements are highly inferior to translation control elements of plastid origin (Herz et al., 2005). However, the translation control element of gene 10 from the coliphage T7 (T7G10) mediates very high expression levels in plastids (Kuroda & Maliga, 2001 b; Herz et al. 2005). Together with translation control elements derived from the highly expressed plastid genes *rbcL* and *psbA,* these are the most commonly used translation control elements for recombinant gene expression in higher plant plastids. Indeed, over 90 % of all recombinant genes expressed in plastids of higher plants published until the end of 2006 used translation control elements derived from T7G10, *rbcL* or *psbA* (see table 8; in Koop et al., 2007). Currently only the T7G10 translation control element fulfills the requirement of stability (no recombination with endogenous elements) and efficiency (mediates high expression levels of the operably linked gene). As such this makes it impossible to express more than one introduced gene stably at high levels.

**Table 1. Regulatory elements used in higher plant plastid transformation**

| Promoters |
|---|
| 16S rRNA (Svab and Maliga 1993) |
| *psbA* (Staub and Maliga 1993) |
| T7G10^{a,b} (McBride et al. 1994) |
| *clpP* (Sriraman et al. 1998) |
| *trc^{a}* (Newell et al. 2003) |
| *rbcL* (Herz et al. 2005) |
| PHS^{a,c} (Buhot et al. 2006) |

| **Translation control elements** |
|---|
| rbcL (rbs) (Svab and Maliga 1993) |
| psbA (Staub and Maliga 1993) |
| T7G10^{a} (Staub et al. 2000) |
| atpB (Kuroda and Maliga 2002) |
| clpP (Kuroda and Maliga 2002) |
| rpl22 (Herz et al. 2005) |
| psbC (Herz et al. 2005) |
| psaB (Herz et al. 2005) |
| PVXcp^{a} (Herz et al. 2005) |
| IRESmp75^{a} (Herz et al. 2005) |
| IREScp148^{a} (Herz et al. 2005) |

| 3'-untranslated regions |
|---|
| psbA (Staub and Maliga 1993) |
| rps16 (Zoubenko et al. 1994) |
| rbcL (Eibl et al. 1999) |
| rpl32 (Eibl et al. 1999) |
| rrnB (Newell et al. 2003) |
| Ta ^{a} (Buhot et al. 2006) |

| |
|---|
| ^{a} Regulatory elements of non-plastid origin: trc (E.coli), PHS (E.coli groE heat shock promoter), T7G10 (phage T7 gene 10 promoter resp. translation control element), IREScp148 (internal ribosomal entry site of the coat protein of a crucifer-infecting tobamovirus), IRESmp75 (internal ribosomal entry site of the movement protein of a crucifer-infecting tobamovirus), PVX (translation control element of the coat protein of Potato virus X), Ta (E.coli threonine attenuator) ^{b} T7-RNA polymerase needed ^{c} Chimeric transcription factor needed |

Therefore, it is an object of the invention to provide a translation control element for expressing a sequence of interest in plant plastids or bacteria. It is another object of the invention to provide translation control elements with a low risk of recombination with endogenous host sequences of plant plastids. It is a further object to provide vectors for expressing gene(s) of interest in plant plastids and bacteria. It is a further object to provide vectors which allow transformation and expression of gene(s) of interest in plastids of higher plants and in bacteria. It is a further object to provide transgenic plants containing translation control elements for efficient and stable expression of gene(s) of interest.

### SUMMARY OF THE INVENTION

The present invention provides a nucleic acid comprising a translation control element and, downstream thereof, a non-yersinia phage coding sequence to be expressed in plant plastids or bacteria, said translation control element comprising
(i) a sequence of SEQ ID NO: 1; or
(ii) a sequence of SEQ ID NO: 2; or
(iii) a sequence having a sequence identity of at least 75 % to the sequence of SEQ ID NO: 1 or 2;
or a complementary nucleic acid having a sequence complementary to that of said nucleic acid.

The invention also provides a plant, plant tissue or plant cells comprising in plastids thereof a nucleic acid comprising a translation control element and, downstream thereof, a coding sequence to be expressed in plastids of said plant, plant tissue or plant cells, said translation control element comprising
(i) a sequence of SEQ ID NO: 1; or
(ii) a sequence of SEQ ID NO: 2; or
(iii) a sequence having a sequence identity of at least 75 % to the sequence of SEQ ID NO: 1 or 2;
or a complementary nucleic acid having a sequence complementary to that of said nucleic acid.

The invention further provides a vector comprising said nucleic acid and a bacterial cell, a plant cell and a plant comprising said nucleic acid or said vector. Further provided is a process of expressing a coding sequence of interest in plastids of a plant, plant tissue or plant cells and a process of producing transplastomic plants, plant tissue or plant cells, said processes comprising providing a plant, plant tissue or plant cell with said nucleic acid or said vector of the invention. The invention also provides a use of a nucleic acid comprising a nucleic acid sequence selected from the group consisting of SEQ ID NO: 1, SEQ ID NO: 2, SEQ ID NO: 3, and a sequence having a sequence identity of at least 75 % to the sequence of SEQ ID NO: 1 or 2 for expressing a sequence of interest in a plant, in plant tissue, in a plant cell, or in bacteria. SEQ ID NOs 1 to 3 are defined in the Annex.

The inventors of the present invention have surprisingly identified further translation control elements of non-plant plastid origin that are similarly efficient as the translation control element from bacteriophage T7 for mediating translation of a downstream coding sequence to be expressed in plant plastids. The invention is surprising, since other translation control elements from bacteriophages tested herein provide a marginal translation efficiency of a downstream coding sequence of interest in plant plastids. The invention provides for the first time the possibility to efficiently express two different coding sequences in plant plastids using two different translation control elements, whereby the risk of recombination between the translation control elements is low and the stability of the transplastome is thus high.

The nucleic acid of the invention contains the translation control elements as defined in the claims. In one embodiment, said translation control element comprises a sequence of SEQ ID NO: 1. In another embodiment, said translation control element comprises a sequence of SEQ ID NO: 2. In a further embodiment, said translation control element comprises a sequence having a sequence identity of at least 75 % to the sequence of SEQ ID NO: 1 or 2. In a further embodiment, said translation control element comprises a sequence having a sequence identity of at least 79 % to the sequence of SEQ ID NO: 1 or 2. In a further embodiment, said translation control element comprises a sequence having a sequence identity of at least 85 % to the sequence of SEQ ID NO: 1 or 2. In a further embodiment, said translation control element comprises a sequence having a sequence identity of at least 90 % to the sequence of SEQ ID NO: 1 or 2. In a further embodiment, said translation control element comprises a sequence having a sequence identity of at least 95 % to the sequence of SEQ ID NO: 1 or 2.

Herein, sequence identities are calculated with AlignX, a component of Vector NTI Suite 7.1, InforMax Inc. using standard settings (K-tuple size: 2; number of best diagonals: 4; window size 4; gap penalty 5; gap opening penalty 15; gap extension penalty 6.66).

The translation control element of the invention comprises a ribosome binding site close to its 3' end. The ribosome binding site may be derived from a native plant plastid gene. The sequence of the translation control element at the ribosome binding site may comprise nucleotide sequence AGGAG, or nucleotide sequence AGGAGG, or nucleotide sequence AGGAGA, or nucleotide sequence AAGGAG, or nucleotide sequence AAGGAGA, or nucleotide sequence AAGGAGAT, or nucleotide sequence AAGGAGATT. In another embodiment, the sequence of the translation control element at the ribosome binding site is selected from the group consisting of nucleotide sequences AAGGAG, AAGGAGA, and AAGGAGAT. In a further embodiment, the sequence of the translation control element at the ribosome binding site of the translation control element of the invention comprises nucleotide sequence AAGGAGATT.

In one embodiment, said translation control element of the invention differs in a sequence segment of 20 bases upstream of the start codon of said coding sequence by at least 5, at least 6, or at least 7 base differences from the sequence segment of 20 bases upstream of the start codon ATG of SEQ ID NO: 39. In another embodiment, said translation control element of the invention differs in a sequence segment of 40 bases upstream of the start codon of said coding sequence by at least 10, at least 12, or at least 14 base differences from the sequence segment of 40 bases upstream of the start codon ATG of SEQ ID NO: 39. The number of base differences is the sum of base substitutions, base additions, and base deletions in an optimum alignment of said 20 or 40 base sequence segments, respectively.

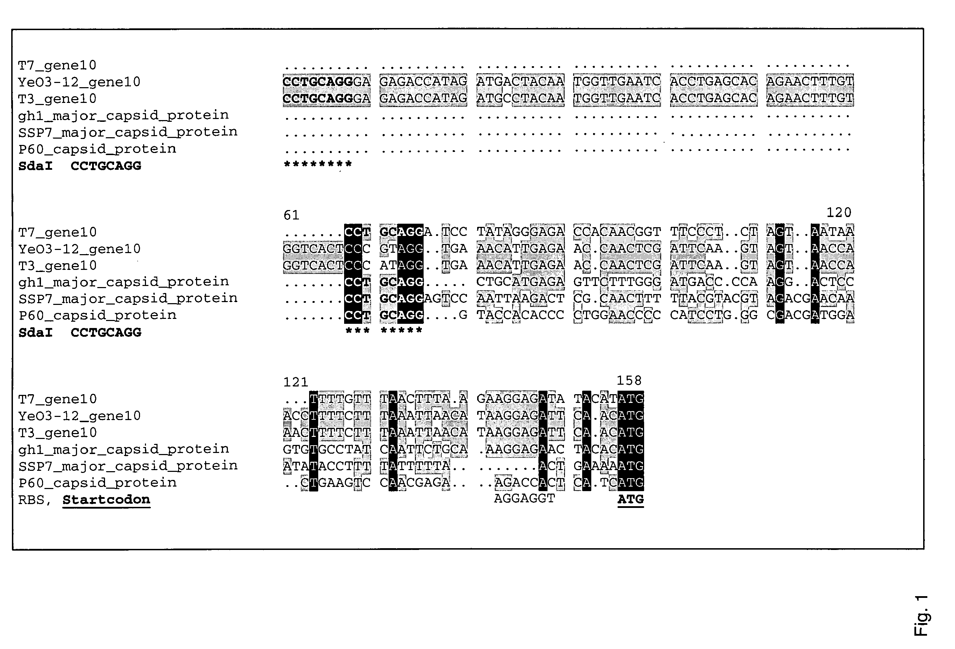

Thus, the invention provides a nucleic acid comprising a translation control element and, downstream thereof, a preferably non-yersinia phage coding sequence to be expressed in plant plastids or bacteria, said translation control element comprising a nucleotide sequence that differs in a sequence segment of 20 bases upstream of the start codon of said coding sequence by at least 5, at least 6, or at least 7 base differences from the sequence segment of 20 bases upstream of the start codon ATG of SEQ ID NO: 39 shown in Fig. 1, top. The invention also provides a nucleic acid comprising a translation control element and, downstream thereof, a preferably non-yersinia phage coding sequence to be expressed in plant plastids or bacteria, said translation control element comprising a nucleotide sequence that differs in a sequence segment of 40 bases upstream of the start codon of said coding sequence by at least 10, at least 12, or at least 14 base differences from the sequence segment of 40 bases upstream of the start codon ATG of SEQ ID NO: 39. These embodiments may of course be combined with other embodiments defined herein such as with the embodiments of the sequences of the translation control element at the ribosome binding site defined above.

The invention further provides a nucleic acid comprising a translation control element and, downstream thereof, a preferably non-yersinia phage coding sequence to be expressed in plant plastids or bacteria, said translation control element differing in its 20 base 3'-terminal sequence segment upstream of the start codon of said coding sequence by at most 5, at most 4, at most 3, or at most 2 base differences from the 20 base 3'-terminal sequence segment of SEQ ID NO: 1. The invention further provides a nucleic acid comprising a translation control element and, downstream thereof, a preferably non-yersinia phage coding sequence to be expressed in plant plastids or bacteria, said translation control element differing in its 40 base 3'-terminal sequence segment upstream of the start codon of said coding sequence by at most 10, at most 8, at most 5, or at most 3 base differences from the 20 base 3'-terminal sequence segment of SEQ ID NO: 1 The number of base differences is the sum of base substitutions, base additions, and base deletions in an optimum alignment of said 20 and 40 base sequence segment, respectively. These embodiments may of course be combined with other embodiments defined herein such as with the embodiments of the sequences of the translation control element at the ribosome binding site defined above.

Downstream and adjacent to the 3' end of the translation control element, the nucleic acid of the invention comprises a coding sequence to be translated. In one embodiment, the nucleic acid of the invention comprises a 3' untranslated region downstream of the coding sequence. Said coding sequence codes for a polypeptide to be produced. Said coding sequence comprises a start codon and, usually, a stop codon at the 3' end of said coding sequence. Said coding sequence may code for any polypeptide of interest. Examples of proteins that may be enocoded by said coding sequence are given below. In one embodiment, said coding sequence does not code for a Yersinia phage protein.

The nucleic acid of the invention may be DNA or RNA. In an embodiment where the nucleic acid is DNA, expression of the coding sequence of the invention requires transcription of the translation control element, the coding sequence, and, optionally, a 3' untranslated region of the DNA nucleic acid in a plant or bacterial host cell. Transcription is typically performed by an RNA polymerase present in the host cell. Transcription may involve a transcription promoter located 5' to said translation control element on said the nucleic acid of the invention. Alternatively, said nucleic acid may be devoid of a transciption promoter and may be placed under the control of a host promoter by transforming said nucleic acid in a plant or bacterial host cell. Thus, said nucleic acid may but does not have to contain a transciption promoter 5' to said translation control element of the invention.

The nucleic acid of the invention may have a second coding sequence to be expressed and upstream thereof a second translation control element. Said second translation control element preferably differs in sequence from said translation control element of the invention in order to minimise the risk of recombination between the translation control elements. Said second translation control elements may have a sequence identity of at most 85 %, preferably at most 75%. In one embodiment, said second translation control element is a bacteriophage T7G10 translation control element.

The invention is not limited regarding the transcription promoter to be used for expressing a coding sequence from said nucleic acid. Any transcription promoter known to be functional in plant plastids may be used for expression in plant plastids and may be combined with the translation control elements of the invention. Examples for promoters that may be used in the invention are given in table 1. However, promoter and translation control elements both influence the expression yield of the coding sequence of interest.

Plastid transformation protocols have been developed for a large variety of different plants including higher (multi-cellular) plants, cf. Maliga, 2004. Plastid transformation protocols have been developed both for many monocotyledonous plants and for many dicotyledonous plants. Thus, the plants, plant tissue, plant cells and processes of the invention are not limited to specific plant species. In one embodiment, dicotyledonous plants, plant tissue and plant cells are used for practicing the present invention.

The nucleic acid of the invention can also be used for expressing a coding sequence in bacteria such as gram-positive and gram-negative bacteria.

The vector of the invention is a transformation vector, notably a plant transformation vector for plant plastids. For plastid transformation, the vector contains the nucleic acid of the invention and homologous flanks for integration of said nucleic acid into plastomes by homologous recombination. Processes of plastid transformation of plants including higher plants is known and is also described in the examples of the present patent application.

### DEFINITIONS

- **3'-UTR:**: transcribed but untranslated region of a (->) **gene,** downstream of a (->) **coding sequence;**
- **5'-UTR:**: transcribed but untranslated region of a (->) **gene,** upstream of a (->) **coding sequence;** in (->) **plastid** (->) **genes,** the **5'-UTR** is referred to herein as translation control element and contains sequence information for translation initiation (ribosome binding site, (->) **RBS)** close to its 3' end;
- **aadA:**: (->) **coding region** of bacterial aminoglycoside adenyl transferase, a frequently used protein, that detoxifies antibiotic (->) **selection inhibitors** spectinomycin and/or streptomycin;
- **aphA-6**: (->) **coding region** of bacterial aminoglycoside phosphotransferase A-6, a protein that detoxifies the antibiotic (->) **selection inhibitor**: kanamycin
- **chloroplast**:: (->) **plastid** containing chlorophyll;
- **coding sequence**:: nucleotide sequence containing the information for an amino acid sequence of a polypeptide; a coding sequence has a start codon at its 5' end; coding sequences are optionally interrupted by one or more (->)**intron**(s);
- **flank, flanking region**:: DNA sequences at the 5' and 3' ends of inserts in a conventional (->) **plastid** (->) **transformation** (->) **vector,** which mediate integration into the target (->) **plastome** of sequences between the flanks by double reciprocal (->) **homologous recombination.** Further, by (->) **homologous recombination,** sequences can be modified or removed from the target (->) **plastome.** Thus, the flanks of the (->) **plastid** (->) **transformation** (->) **vector** determine, where changes in the target (->) **plastome** are generated by (->) **transformation;**
- **gene expression:**: process turning sequence information into function; in (->) **genes** encoding polypeptides, gene expression requires the activity of a (->) **promoter,** which initiates and directs RNA polymerase activity, leading to the formation of a messenger RNA (mRNA), a coding sequence of which is subsequently translated into a polypeptide; in (->) **genes** encoding RNA, the (->) **promoter-**mediated activity of RNA polymerase generates the encoded RNA;
- **gene(s)**:: nucleotide sequence(s) encoding all elements, which are required to secure function e.g. expression;
genes are organised in (->) **operons** which contain at least one complete (->) **coding sequence**; in (->) **genes** encoding polypeptides, these elements are: (1) a (->) **promoter**, (2) a 5' untranslated region ((->) **5'-UTR),** (3) a complete (->) **coding sequence***,* (4) a 3' untranslated region ((->) **3'-UTR)**; in (->) **genes** encoding RNA, the (->) **5'-UTR** and the (->) **3'-UTR** are missing; in (->) **operons** having more than one (->) **coding sequence***,* two subsequent complete (->) **coding sequences** are separated by a (->) **spacer***,* and (->) **promoter***,* (->) **5'-UTR,** and (->) **3'-UTR** elements are shared by the **(->)coding regions** of that (->)**operon;**
- **gene of interest:**: modified or newly introduced gene: the purpose of a (->) **transformation** attempt;
- **genome**:: Complete DNA sequence of a cell's nucleus or a cell organelle;
- **GFP**: green fluorescent protein
- **homologous recombination**:: process leading to exchange, insertion or deletion of sequences due to the presence of one or more (->) **flanks** with sufficient sequence homology to a target site in a (->) **genome;**
- **host or host cell**:: organism wherein the coding sequence of the invention is to be expressed; herein, said host is a plant or a bacterium; accordingly, said host cell is a plant cell or a bacterial cell;
- **intron:**: sequence interrupting a (->) **coding region;**
- **operon:**: organisational structure of one or more(->) **genes** sharing a promoter;
- **plant(s):**: organism(s) that contain(s) (->) **plastids** in its (their) cells; this invention particularly relates to multicellular (->) **plants;** these include the group of gymnosperms (such as pine, spruce and fir etc.) and angiosperms (such as the *monocotyledonous* crops maize, wheat, barley, rice, rye, Triticale, sorghum, sugar cane, asparagus, garlic, palm tress etc., and non-crop monocots, and the *dicotyledonous* crops tobacco, potato, tomato, rape seed, sugar beet, squash, cucumber, melon, pepper, Citrus species, egg plant, grapes, sunflower, soybean, alfalfa, cotton etc.), and no-crop dicots as well as ferns, liverworths, mosses, and multicellular green, red and brown algae;
- **plastid(s):**: organelle(s) with their own genetic machinery in (->) **plant** cells, occurring in various functionally and morphologically different forms, e.g. amyloplasts, (->) **chloroplasts,** chromoplasts, etioplasts, gerontoplasts, leukoplasts, proplastids etc;
- **plastome:**: complete DNA sequence of the (->) **plastid;**
- **promoter:**: nucleotide sequence functional in initiating and regulating transcription;
- **RBS, ribosomal binding site:**: DNA sequence element upstream of the (->) **translation start codon** of a (->) **coding sequence,** that mediates ribosome binding and translation initiation from the respective RNA transcript; **RBS** elements are either part of (->) **5=-UTRs** or of (->) spacers;
- **selection inhibitor:**: chemical compound, that reduces growth and development of non-transformed cells or organelles stronger than that of transformed ones;
- **sequence of interest**: modified or newly introduced sequence: the purpose of a (->) **transformation** attempt;
- **spacer:**: transcribed but not translated region of a (->) **gene,** located between two adjacent (->) **coding sequences** of an polycistronic **(->)operon;** in (->) **plastid** (->) **genes,** the **spacer** is a **translation control element** and contains sequence information for translation initiation (ribosome binding site, (->) **RBS)** close to its 3' end;
- **transformation vector:**: cloned DNA molecule that was generated to mediate (->) **transformation** of a (->) **genome;**
- **transformation:**: process leading to the introduction, the excision or the modification of DNA sequences by treatment of (->) **hosts** or (->) **host cells** including the use of at least one (->) **transformation vector**;
- **transgene:**: DNA sequence derived from one (->) **genome**, introduced into another one;
- **translation control element:**: (->) **5'-UTR** or (->) **spacer;** transcribed but not translated region of a (->) **gene,** upstream of a (->) **coding sequence;** in (->) **plastid** (->) **genes,** the **translation control element** contains sequence information for translation initiation (ribosome binding site, (->) **RBS)** close to its 3' end;
- **uidA:**: (->) **coding region** of bacterial β glucuronidase, a frequently used reporter protein.

### SHORT DESCRIPTION OF THE FIGURES

**Figure 1** Alignment of translation control elements from different phages. Sequences are shown from the artificial Sdal restriction enzyme recognition site (used for cloning) to the start codon of the reporter gene. The sequences shown are referred to as SEQ ID NO: 39 to SEQ ID NO: 44 in the order shown.
**Figure 2** Coomassie stained SDS-PAGE of plant raw extract from transplastomic plants harbouring translation control elements from different phages.
   1. Plant line 904-4-1 (P-SSP7)
   2. Plant line 904-3-1 (P-SSP7)
   M Molecular Weight Marker (117 kDa, 90 kDa, 49 kDa, 35 kDa)
   3. Wildtype
   4. Plant line 899-8-2 (φYeO3-12)
   5. Plant line 714-9-1 (T7)
   6. Plant line 894-8-1 (gh1)
   7. Plant line 906-4-1 (gh1)
   8. Plant line 903-5-1 (P60)
   9. Plant line 892-9-1 (P60)
   The arrow depicts the GUS reporter protein.

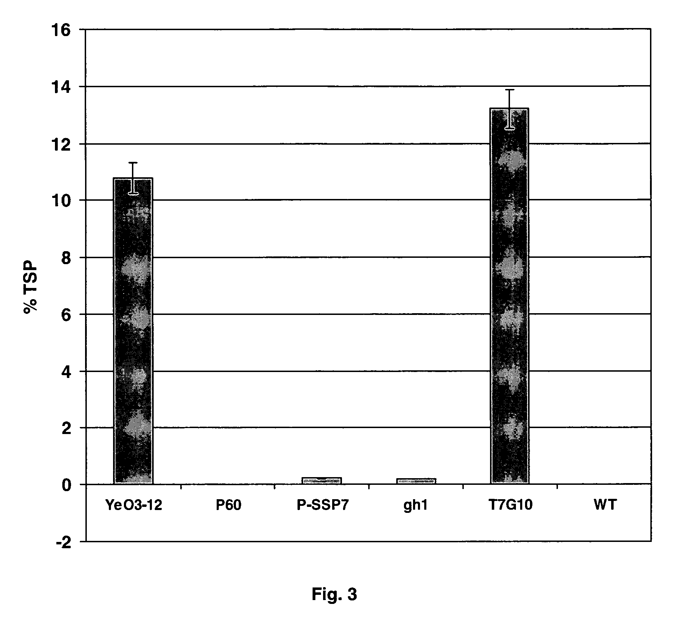

**Figure 3** Photometric GUS activity assay (conversion of nitro-phenyl-glucuronide into p-nitrophenol) with-5 µl of 1:100 diluted raw extract from transplastomic plants with translation control elements from different phages. The percentage of GUS in the total soluble protein (% TSP) is calculated from the measured kinetic GUS activity in the samples and a GUS-standard (Sigma); the protein concentration in the samples was determined by Bradford.

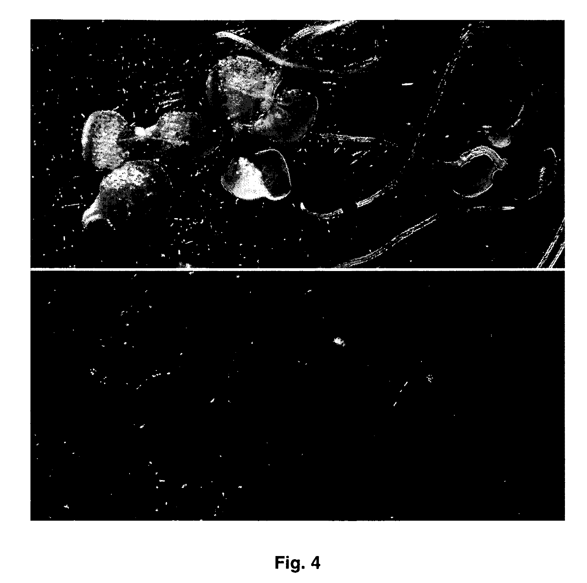

**Figure 4** GUS staining of seedlings of wild type and transformed plant lines. Upper panel shows wild type seedlings after staining and treatment with 70% ethanol to remove the chlorophyll. Wild type seedlings are colorless. In the lower panel seedlings of a transformed plant line are shown. GUS-activity leads to a dark blue color of the seedlings after staining and ethanol treatment.

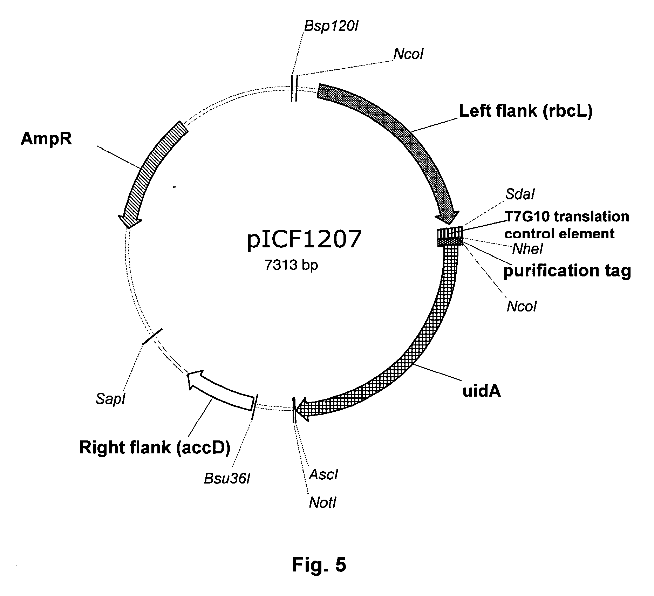

**Figure 5** Schematic representation of vector pICF1207. The uidA-gene together with the control element T7 and a purification tag are inserted between the *rbcL* and the *accD* gene of the plastome, downstream of the *rbcL* gene and upstream of the rbcL-terminator.

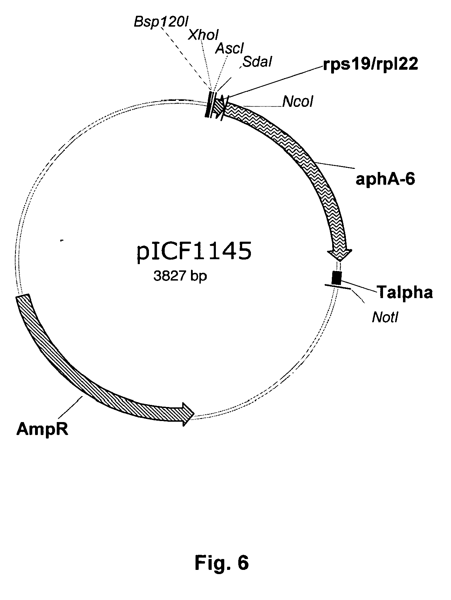

**Figure 6** Schematic representation of vector pICF1145. The vector carries a selection cassette comprising the translation control element rps19/rpl22, the *aphA-6* gene, and the alpha-terminator of *E.coli.* The *aphA-6* gene confers resistance to kanamycin.

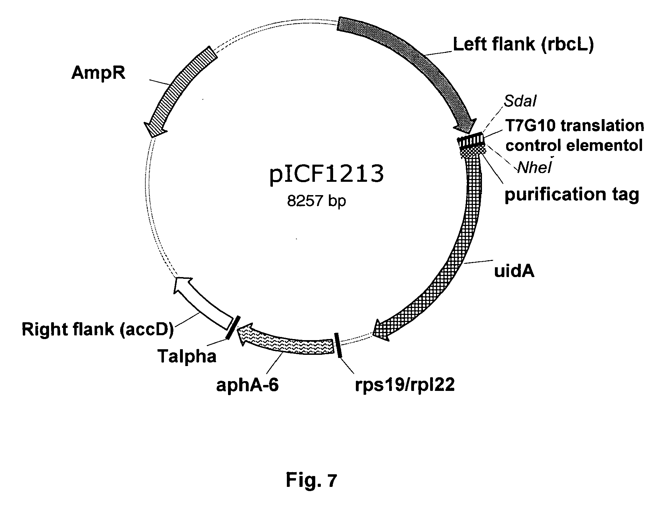

**Figure 7** Schematic representation of vector pICF1213. Plastid transformation vector for insertion of the *uidA*-gene, purification tag and translation control element T7 downstream of the *rbcL*-gene, conferring stable integration of the *aphA-6* selection cassette into the plastome.

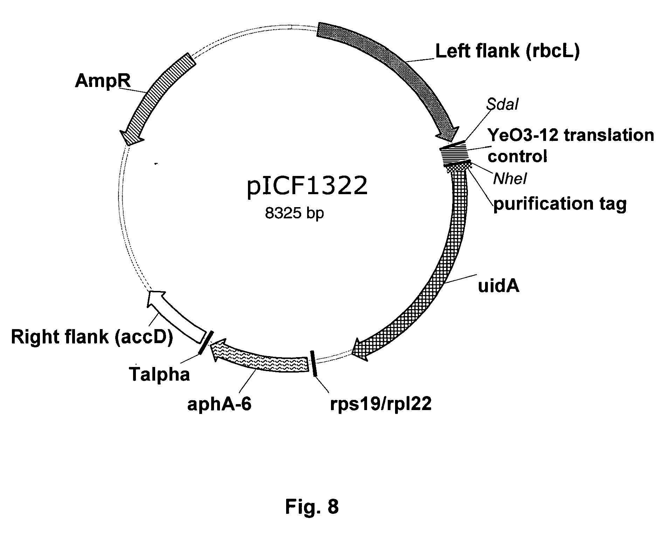

**Figure 8** Schematic representation of vector pICF1322. Plastid transformation vector for insertion of the *uidA*-gene, purification tag and translation control element YeO3-12 downstream of the *rbcL*-gene, conferring stable integration of the *aphA-6* selection cassette into the plastome.

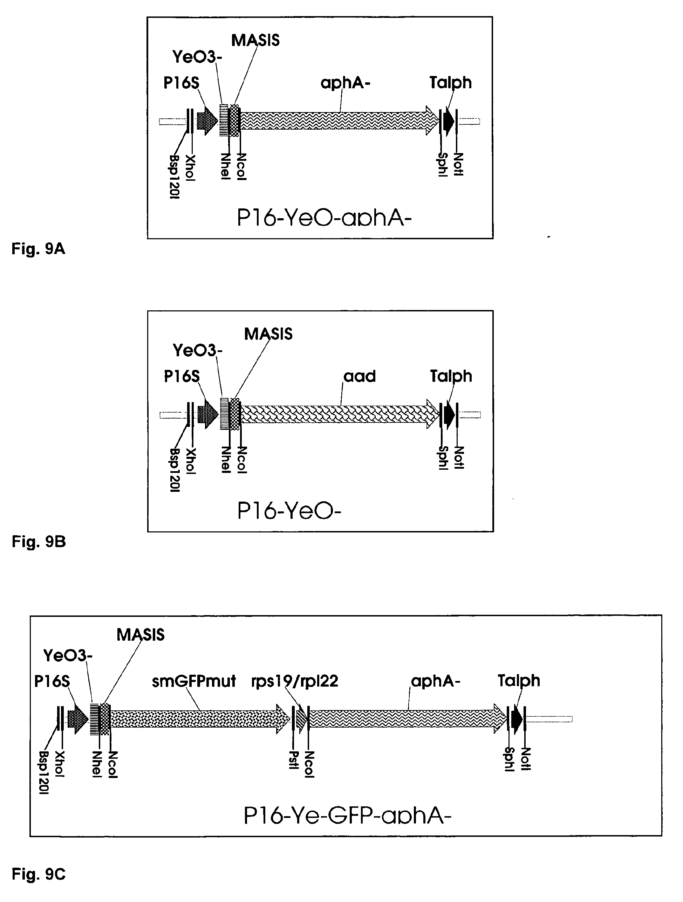

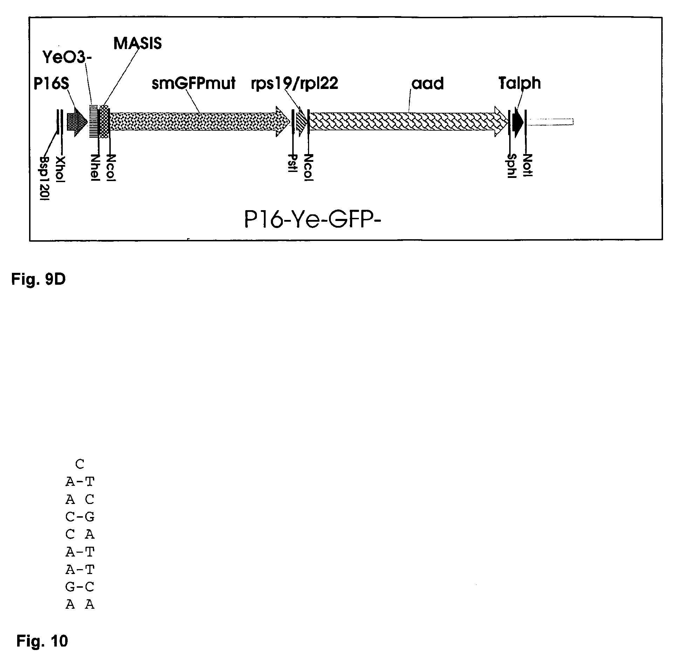

**Figure 9** Schematic views of selection cassettes using the translation control element YeO3-12. **A** Selection cassette consisting of 16S-promoter, translation control element YeO3-12, selection gene *aphA-6* with synthetic N-terminus MASIS, conferring resistance to spectinomycin and the alpha terminator of *E.coli.* **B** Selection cassette consisting of 16S-promoter, translation control element YeO3-12, selection gene *aadA* with synthetic N-terminus MASIS, conferring resistance to kanamycin and the alpha terminator of *E.coli.* **C** Selection cassette consisting of 16S-promoter, translation control element YeO3-12, visible marker gene smGFP with synthetic N-terminus MASIS, translation control element rps19/rpl22, selection gene *aphA-6* conferring resistance to spectinomycin and the alpha terminator of *E.coli.* **D** Selection cassette consisting of 16S-promoter, translation control element YeO3-12, visible marker gene smGFP with synthetic N-terminus MASIS, translation control element rps19/rpl22, selection gene *aadA* conferring resistance to kanamycin and the alpha terminator of *E.coli.*
**Figure 10** Potential stem-loop structure in translation control element YeO3-12 (SEQ ID NO: 45).

### DETAILED DESCRIPTION OF THE INVENTION

The translation control element of the phage T7 gene 10 controls the expression of the major capsid protein of the phage. It is a suitable control element for heterologous expression of genes of interest in the bacteria *Escherichia coli.* It is also a suitable control element for expression of genes of interest in plant plastids. Phage T7 is a coliphage (member of the Podoviridae) infecting gram negative bacteria. There are other phages belonging to the Podoviriadae which infect cyanobacteria, (Sullivan et al., 2005). Cyanobacteria are the closest relatives to plant plastids. Therefore, we tested whether translation control elements of the major capsid protein from cyanophages would be similar or even more suitable for expression of genes of interest in plant plastids than the T7G10 element. We chose the translation control elements of the major capsid protein from the cyanophages P-SSP7 and P60, both members of the T7-supergroup (Chen & Schneider, 2005) to assay their efficiency in plastid expression vectors. We used translation control elements of the major capsid protein from two other members of the T7 supergroup phages as controls, the pseudomonas phage gh-1 and the yersiniophage φYeO3-12 (Fig. 1). All these translation control elements were inserted upstream of a GUS reporter gene in otherwise identical plastid transformation vectors and compared to a similar vector containing the T7G10 translation control element. The GUS expression level mediated by the different translation control element was assayed by monitoring GUS activity and protein analysis on Coomassie stained SDS-PAGE.

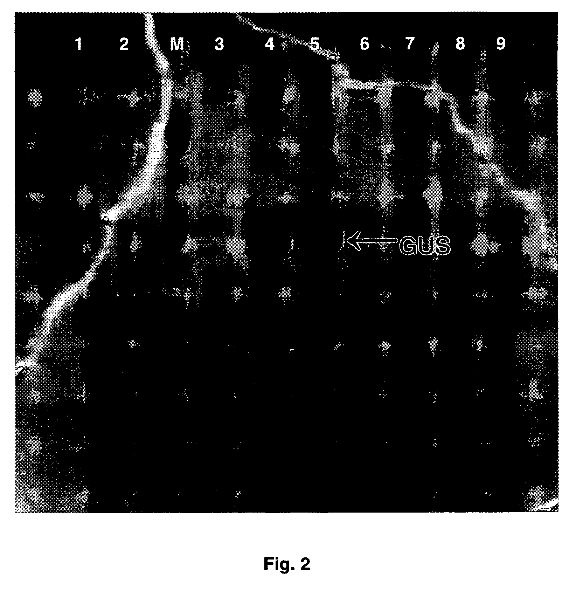

The translation control element from cyanophage P60 mediates an almost undetectable GUS expression level whereas the elements from cyanophage P-SSP7 and pseudomonas phage gh-1 mediate low expression levels. Surprisingly, it was found that the translation control element from yersinophage φYeO3-12 mediates a very high expression level in plant plastids (Figures 2 and 3).

The translation control element from yersiniophage φYeO3-12 can be used to direct expression a gene of interest in plastids. For that purpose, it may be operably linked to an upstream promoter and a downstream coding sequence. A simple embodiment is an expression cassette comprising in 5' to 3' direction: a promoter, the translation control element of the invention such as that from yersiniophage φYeO3-12, the coding sequence of the gene of interest and a 3'-UTR. The translation control element from yersiniophage φYeO3-12 acts as 5'-UTR in this embodiment. Examples of suitable promoters and 3'-UTRs can be found in table 1. In other embodiments, the translation control element from yersiniophage φYeO3-12 is used in polycistronic operons and controls translation of a downstream coding sequence. Hence, the promoter and the translation control element from yersiniophage φYeO3-12 may be separated by one ore more preceding genes. In this case, the translation control element from yersiniophage φYeO3-12 may act as spacer element. The coding sequence under translational control of the translation control element of the invention may also be followed by additional genes, transcribed from the same promoter.

The translation control element of the inventiontogether with the translation control element from coliphage T7 enables stable, simultaneous and strong expression of two heterologous proteins in plastids. This is extremely valuable for heterooligomeric (e.g. heterodimeric) proteins like antibodies. Antibody-production in plants ("plantibody") is a promising technology to satisfy the increasing demand for therapeutical or diagnostic antibodies.

The translation control element from yersiniophage φYeO3-12 is not only useful for the expression of heterologous genes in plastids but also in bacterial hosts like Escherichia coli.

This invention describes new translation control elements. Together with a promoter, a translation control element enables expression of a gene of interest. While the promoter directs the transcription of DNA into mRNA, the translation control element directs translation of the coding sequence within the mRNA into a protein or peptide. Whereas one promoter can direct expression of many genes in a polycistronic operon, every coding sequence needs its own translation control element for translation into a protein or peptide.
A translation control element contains a sequence or sequences necessary for ribosome binding, the so called ribosome binding site, often also referred to as the Shine-Dalgarno-Sequence. Additionally, it may contain further elements to improve RNA-stability and translation. Such further elements can be structural elements e.g. complementary sequences forming loops or hairpins. The translation control element can be a 5'-UTR if it is located at the 5'-end of the transcript or a spacer element if it is located between two adjacent genes. In every case the translation control element is responsible for translation initiation of the downstream coding sequence.

Translation control elements are needed if a new gene is introduced into the genome of a host cell. These translation control elements have to be introduced together with the coding sequence to ensure translation into a protein or peptide. Translation control elements from phages are well suited for bacteria because bacteria are their natural hosts or may be closely related to the natural hosts. It is well known that the translation control element for gene 10 from coliphage T7 provides strong expression of a downstream coding sequence in the host *E.coli* and also in plant plastids. However, many translation control elements from bacteriophages do not provide satisfactory translation efficiencies in plant plastids.

Translation control elements from cyanophages which infect hosts that are even more closely related to plastids than gram negative bacteria like *E.coli* have been found not to be suitable for directing high level expression in plastids . Gene 10 in phage T7 codes for the major capsid protein of this phage. Equivalent translation control element from the cyanophages P60 and P-SSP7 mediates only very low expression levels (see example 1 and figure 3).

Furthermore, Pseudomonas phage gh-1 and yersiniophage φYeO3-12 both belong to the T7 group of phages. Nevertheless, the translation control element from gh-1 directs very low expression of a reporter gene in plant plastids, whereas the translation control element from φYeO3-12 directs very high expression of the same reporter gene. Thus, it cannot be predicted whether a translation control element mediates high expression in plant plastids ,

The translation control element from yersiniophage φYeO3-12 (SEQ ID NO: 1) was found to be exceptionally well suited for translation control of a heterologous coding sequence in plant plastids. This translation control element can for example be used as 5'-UTR in standard expression cassettes comprising promoter, 5'-UTR, coding sequence and 3'-UTR. It can also be used as spacer element in operon extension vectors where an existing plastid operon is extended by one or more cistrons.

The translation control element can also be combined with N-terminal fusion tags downstream of the start codon (e.g. MASIS-element; Herz et al. 2005) to increase the expression level furthermore. The N-terminal fusion tag can also be used to simplify purification of the recombinant protein (e.g. His-tag).

### Use of the translation control element in operon extension vectors

An operon extension vector by definition extends an existing plastid operon by one or more genes, which are then all transcribed collectively from the endogenous plastid operon promoter. No promoter has to be present in the transformation vector. Examples for such vectors are described in Herz et al. 2005. The transformation vector typically comprise in 5' to 3' orientation: (1) a left flanking region comprising part of a plastid operon (e.g. part of the rbcL coding sequence or psbA coding sequence or alike). (2) the translation control element of this invention. (3) a coding sequence of interest (e.g. coding for a reporter protein like β-glucuronidase, a pharmaceutical protein like interferon, a sweetener protein like thaumatin, an antibody component like the heavy chain etc.). (4) a spacer element (e.g. rpI22 or psbC or alike). (5) coding sequence for a selection marker (e.g. from aphA-6 or aadA or alike). (6) a 3'-UTR (e.g. from plastid rbcL or E.coli alpha operon or alike). (7) a right flanking sequence comprising plastid sequence adjacent to the left flanking region.

Such an operon extension vector can contain additional elements for example between (3) and (4) an additional coding sequence (e.g. antibody light chain) under control of an additional translation control element (e.g. from phage T7 gene 10).

### Use of the translation control element in standard expression cassettes

Generally, standard expression cassettes for gene expression in plastids comprise in 5' to 3' orientation: (1) a promoter functional in plastids (e.g. 16S or psbA or alike). (2) the translation control element of this invention (e.g. SEQ ID NO: 1 or SEQ ID NO: 2 or alike). (3) a coding sequence of interest (e.g. coding for a reporter protein like β-glucuronidase, a pharmaceutical protein like interferon, a sweetener protein like thaumatin, an antibody component like the heavy chain etc.). (4) a 3'-UTR (e.g. from plastid rbcL or E.coli alpha operon or alike).

Such an expression cassette can contain additional elements, for example between (3) and (4) an additional coding sequence (e.g. a selection marker like aphA-6 or a second protein component like an antibody light chain etc.) under control of an additional translation control element (e.g. from rpl22 or T7G10).

The expression cassette is usually flanked by sequences homologous to the desired plastome insertion site to facilitate stable insertion via double homologous recombination. A wide range of insertion sites have been used for plastid transformation (reviewed by Maliga 2004). Some flanking regions cloned from one species can be used for transformation of other species because of their high inherent homology. Indeed, the concept of a universal vector has been described (WO9910513) allowing direct transformation of a wide range of dicotyledonous and monocotyledonous plant species. Placing the described expression cassette between such flanking regions allows vectors containing the translation control sequence of this invention to be used for the transformation of and expression in a wide range of plant species. Nevertheless, the expression cassette can also be placed outside of the flanking sequences in the vector backbone (described in detail in Klaus et al. 2004; Koop et al. 2007, or WO03/004658). In this case, the expression cassette is transiently inserted into the plastome. Such an arrangement is especially useful if the selection and/or reporter genes are not required in the final transplastomic plant. GFP is especially useful as a reporter protein because transformed tissue can be visualized at a very early stage under a fluorescence microscope. For that purpose, it is preferred to place GFP under control of a very strong translation control element. However, usually the gene of interest within the homologous flanks also needs a strong translation control element. With the translation control elements of the invention and that of coliphage T7 it is now for the first time possible to use two powerful non-plastid control elements within one vector for mediating high expression of two or more different coding sequences. The same is true, if high expression is desired for the gene of interest and also for a transient or stable selection gene.

### Use of the translation control element in bacteria

Some plastid promoters like the 16S promoter are also able to function in bacterial hosts like E. coli. The same is true for some translation control elements like the translation control elements for the major capsid protein from yersiniophage φYeO3-12 (SEQ ID NO: 1). Therefore standard expression cassettes as described above can also be used to direct expression of one or more genes of interest in a bacterial host like E. coli. The translation control elements of this invention can of course also be combined with standard bacterial promoters and operators like the lac-promoter/operator system.

### Use of similar or modified translation control elements

A database search with the translation control element from yersiniophage φYeO3-12 (SEQ ID NO: 1) revealed a very similar translation control element from the Coliphage T3 (Fig. 1). Both translation control elements differ only by 3 bp. The translation control element from coliphage T3 is also a very suitable element for expression cassettes in plastids.
The translation control elements from φYeO3-12 and T3 are approximately twice as long as the translation control elements from T7, gh1, SSP7 and P60 (Fig. 1). For some applications, it is useful if compact elements are available. A truncated translation control elements from φYeO3-12 comprising only 68 bp upstream of the start codon (SEQ ID NO: 2) can also be used to mediate expression of a gene of interest in plastids. This core sequence shows very high identity to the equivalently truncated T3 sequence, but low identity to the truncated elements from T7, gh1, SSP7 and P60 (Table 2).

**Table 2. Identity of translation control core sequences (comprising 68 bp upstream of the start codon)**

| | φYeO3-12 | T7 |
|---|---|---|
| φYeO3-12 | 100 % | 56.9 % |
| T7 | 56.9 % | 100 % |
| T3 | 98.5 % | 56.9 % |
| P60 | 42.3 % | 41.9 % |
| SSP7 | 45.9 % | 45.7 % |
| gh1 | 45.7 % | 48.7 % |
| SEQ ID NO. 3 | 79.4 % | 58.3 % |

% identity was calculated with AlignX, a component of Vector NTI Suite 7.1, InforMax Inc. as described above.

Further modification of the φYeO3-12 core sequence may simplify cloning and assembly of the expression cassette e.g. by introducing additional recognition sites for restriction enzymes. Sequence modification of the φYeO3-12 core sequence may introduce new characteristics (e.g. extended codon-anticodon interaction by changing C->T at position -1) or remove existing characteristics like stem-loops (Fig. 10) etc. An example for such a modified translation control element is given in SEQ ID NO: 3 given in the Annex.

Proteins of interest, or fragments thereof, that can be expressed using the invention include: starch modifying enzymes (starch synthase, starch phosphorylation enzyme, debranching enzyme, starch branching enzyme, starch branching enzyme II, granule bound starch synthase), sucrose phosphate synthase, sucrose phosphorylase, polygalacturonase, polyfructan sucrase, ADP glucose pyrophosphorylase, cyclodextrin glycosyltransferase, fructosyl transferase, glycogen synthase, pectin esterase, aprotinin, avidin, bacterial levansucrase, *E*. *coli* glgA protein, MAPK4 and orthologues, nitrogen assimilation/methabolism enzyme, glutamine synthase, plant osmotin, 2S albumin, thaumatin, site-specific recombinase/integrase (FLP, Cre, R recombinase, Int, SSVI Integrase R, Integrase phiC31, or an active fragment or variant thereof), isopentenyl transferase, Sca M5 (soybean calmodulin), coleopteran type toxin or an insecticidally active fragment, ubiquitin conjugating enzyme (E2) fusion proteins, enzymes that metabolise lipids, amino acids, sugars, nucleic acids and polysaccharides, superoxide dismutase, inactive proenzyme form of a protease, plant protein toxins, traits altering fiber in fiber producing plants, Coleopteran active toxin from *Bacillus thuringiensis* (Bt2 toxin, insecticidal crystal protein (ICP), CrylC toxin, delta endotoxin, polyopeptide toxin, protoxin etc.), insect specific toxin AalT, cellulose degrading enzymes, E1 cellulase from *Acidothermus celluloticus,* lignin modifying enzymes, cinnamoyl alcohol dehydrogenase, trehalose-6-phosphate synthase, enzymes of cytokinin metabolic pathway, HMG-CoA reductase, *E. coli* inorganic pyrophosphatase, seed storage protein, *Erwinia herbicola* lycopen synthase, ACC oxidase, pTOM36 encoded protein, phytase, ketohydrolase, acetoacetyl CoA reductase, PHB (polyhydroxybutanoate) synthase, acyl carrier protein, napin, EA9, non-higher plant phytoene synthase, pTOM5 encoded protein, ETR (ethylene receptor), plastidic pyruvate phosphate dikinase, nematode-inducible transmembrane pore protein, trait enhancing photosynthetic or plastid function of the plant cell, stilbene synthase, an enzyme capable of hydroxylating phenols, catechol dioxygenase, catechol 2,3-dioxygenase, chloromuconate cycloisomerase, anthranilate synthase, *Brassica* AGL15 protein, fructose 1,6-biphosphatase (FBPase), AMV RNA3, PVY replicase, PLRV replicase, potyvirus coat protein, CMV coat protein, TMV coat protein, luteovirus replicase, MDMV messenger RNA, mutant geminiviral replicase, *Umbellularia californica* C12:0 preferring acyl-ACP thioesterase, plant C10 or C12:0 preferring acyl-ACP thioesterase, C14:0 preferring acyl-ACP thioesterase (luxD), plant synthase factor A, plant synthase factor B, 6-desaturase, protein having an enzymatic activity in the peroxysomal -oxidation of fatty acids in plant cells, acyl-CoA oxidase, 3-ketoacyl-CoA thiolase, lipase, maize acetyl-CoA-carboxylase, 5-enolpyruvylshikimate-3-phosphate synthase (EPSP), phosphinothricin acetyl transferase (BAR, PAT), CP4 protein, ACC deaminase, ribozyme, protein having posttranslational cleavage site, protein fusion consisting of a DNA-binding domain of Gal4 transcriptional activator and a transcriptional activation domain, a translational fusion of oleosin protein with protein of interest capable of targeting the fusion protein into the lipid phase, DHPS gene conferring sulfonamide resistance, bacterial nitrilase, 2,4-D monooxygenase, acetolactate synthase or acetohydroxyacid synthase (ALS, AHAS), polygalacturonase, bacterial nitrilase, fusion of amino terminal hydrophobic region of a mature phosphate translocator protein residing in the inner envelope membrane of the plastid with protein of interest to be targeted into said membrane etc.

Any human or animal protein can be expressed using the system of the invention. Examples of such proteins of interest include inter alia the following proteins (pharmaceutical proteins): immune response proteins (monoclonal antibodies, single chain antibodies, T cell receptors etc.), antigens, colony stimulating factors, relaxins, polypeptide hormones, cytokines and their receptors, interferons, growth factors and coagulation factors, enzymatically active lysosomal enzyme, fibrinolytic polypeptides, blood clotting factors, trypsinogen, 1-antitrypsin (AAT), as well as function-conservative proteins like fusions, mutant versions and synthetic derivatives of the above proteins.

### EXAMPLES

Molecular biology methods used in this invention are well known in the art and are described for example by Sambrook et al. (1989) Molecular cloning and Ausubel et al. (1999) Short protocols in Molecular Biology.

### Example 1: Construction of reporter vectors based on rbcL operon extension

### Cloning of the flanks for homologous-recombination

Flanks for homologous recombination were amplified from tobacco wild type DNA using oligos o458 5'-gaaagagggcccaataatgatgtatttggc-3' (SEQ ID NO: 4) and o466 5'-ctcgagctcctgcaggtttatctgctaatg-3' (SEQ ID NO: 5) for the left flank and oligos o460 5'-gctcgagctcgcggccgcagataaattagcagg-3' (SEQ ID NO: 6) and o461 5'-catctttccctgcagttttcttgccc-3' (SEQ ID NO: 7) for the right flank. Amplified sequences correspond to bases 57411 - 59057 of the tobacco plastome (Genbank Accession number Z00044) for the left flank and to bases 59051 - 59844 for the right flank. The left flank fragment was cut with Bsp1201 and Sacl, the right flank fragment was cut with SacI and PstI, both fragments were cloned into the pGEM (Promega Corp., USA) restricted with Bsp120I and Nsil to give vector pGEM-rbcL.

### Introduction of a translation control element from phage T7, a purification tag and a reporter gene

Vector pGEM-rbcL was restricted Sdal/Notl and ligated with a linker fragment for the translation control element T7G10 and the MASIS-N-terminal fusion tag (consisting of oligo T7G10for 5'-ggatcctatagggagaccacaacggtttccctctagtaataattttgtttaactttaagaaggagatatacatatggctagcatttc-3' (SEQ ID NO: 8) and oligo T7G10rev 5'-catggaaatgctagccatatgtatatctccttcttaaagttaaacaaaattattactagagggaaaccgttgtggtctccctatagga tcctgca-3' (SEQ ID NO: 9); protruding ends compatible to Sdal and Ncol at the 5'- and the 3'-end, respectively) and a PCR amplified fragment for the coding region of the *uidA-*gene, which was amplified from vector RBS::rbc (Eibl et al., 1999) using primers o74 5'-catgccatggtccgtcctgtagaa-3' (SEQ ID NO: 10) and o98 5'-ctgcggccgcgccggcgcgccggtaccttattgtttgcctccctgctgcg-3' (SEQ ID NO: 11) and restricted with enzymes Ncol and Notl.

The resulting intermediate vector was restricted with Nhel and Ncol and ligated with a linker consisting of the oligos o369 5'- ctagcatttccaagaaaggtggacaccatcaccatcaccatgc-3' (SEQ ID NO: 12) and o370 5'- catggcatggtgatggtgatggtgtccacctttcttggaaatg-3' (SEQ ID NO: 13) introducing a purification tag consisting of 10 amino acids (Lys-Lys-Gly-Gly-His-His-His-His-His-His, (SEQ ID NO: 14)). The resulting vector was sequenced and listed as pICF1207 (for details see Figure 5).

### Construction of the selection cassette rps19-aphA-6-Talpha

An Xhol-Ascl-Sdal linker consisting of oligos o308 5'-ggccctcgaggcgcgcctgcagg-3' (SEQ ID NO: 15) and o309 5'-ggcccctgcaggcgcgcctcgag-3' (SEQ ID NO: 16) was cloned into the Bsp120I site of pBluescript KS II (Stratagene) yielding vector plCF884-5.This vector was restricted with Sdal/Xbal and ligated with the translation control element rps19 (linker fragment consisting of oligos Srps19/rpl22-for 5'-gataaaaaaaatctacatgcttatgattcagtagtaggaggcaaac-3' (SEQ ID NO: 17) and Srps19/rpl22-rev 5'-catggtttgcctcctactactgaatcataagcatgtagattttttttatctgca-3' (SEQ ID NO: 18); protruding ends compatible to Sdal at the 5'-end and Ncol at the 3'-end) and a fragment from vector pSK-kmR (Bateman and Purton, 2000) excised with Ncol/Xbal containing the coding sequence for the kanamycin resistance gene *aphA-6.* Into this intermediate vector plCFrps-aphA-6 the alpha terminator from *E.coli* was introduced. For this purpose, the intermediate vector was restricted with Nhel/Notl and ligated with a linker consisting of oligos Talpha-for 5'-ctagaaggcaacgtaaaaaaacccgccccggcgggtttttttatacccgtagtatccccagc-3' (SEQ ID NO: 19) and Talpha-rev 5'-ggccgctggggatactacgggtataaaaaaacccgccggggcgggtttttttacgttgcctt-3' (SEQ ID NO: 20) producing protruding compatible ends to NheI and NotI. The finished vector was sequenced and listed as pICF1145 (for details see Figure 6).

### Introduction of the selection cassette into vector pICF1207

Vector pICF1207 was restricted with Bsu361 and treated with T4-polymerase and Calf intestine alkaline phosphatase to generate dephosphorylated blunt ends. The selection cassette was excised from vector pICF1145 with Ascl/Notl and treated with T4-polymerase to obtain blunt ends. The selection cassette fragment was ligated into vector pICF1207. The resulting vector with the correct orientation of the selection cassette was named pICF1213 (for details see Figure 7).

### Introduction of translation control elements from different phages into plCF1213

Translation control elements of capsid proteins from 4 different phages were tested for their capability to enhance transgenic protein production in plastids. For comparison studies the translation control element of coliphage T7 was exchanged for elements from other phages in plCF1213.

The T7-element was removed by restriction with Sdal/Nhel. The different translation control elements were ordered as oligos, annealed and restricted with Sdal/Nhel and inserted as linker molecules into pICF1213. For the translation control elements from yersiniaphage YeO3-12 which is twice the length of the other elements, the element was split in two fragments which were joined by a Bpil-restriction site. For the sequences of the translation control elements see adjacent Table 3.

**Table 3**

| | |
|---|---|
| gh1-For | aaCCTGCAGGctgcatgagagttctttgggatgaccccaaggactccgtgtgcctat (SEQ ID NO: 21) |
| gh1-Rev | gaaatGCTAGCcatgtgtagttctcctttgcagaattgataggcacacggagtccttg (SEQ ID NO: 22) |
| P60-For | aaCCTGCAGGgtaccacacccctggaacccccatcctgggcgacgatggactgaag (SEQ ID NO: 23) |
| P60-Rev | gaaatGCTAGCcatgatgagtggtcttctcgttggacttcacgtccatcgtcgccc (SEQ ID NO: 24) |
| SSP-For | aaCCTGCAGGagtccaattaagactcgcaactttttacgtacgtagacgaacaaatat (SEQ ID NO: 25) |
| SSP-Rev | gaaatGCTAGCcatttttcagttaaaaataaaaggtatatttgttcgtctacgtacgt (SEQ ID NO: 26) |
| YeO-For | aaCCTGCAGGgagagaccatagatgactacaatggttgaatcacctgagcacagaac (SEQ ID NO: 27) |
| YeO-BpiR | gGAAGACatgtttcacctacgggagtgaccacaaagttctgtgctcaggtgattca (SEQ ID NO: 28) |
| YeO-BpiF | gGAAGACtgaaacattgagaaccaactcgattcaagtagtaaccaaccttttcttta (SEQ ID NO: 29) |
| YeO-Rev | gaaatGCTAGCcatgttgaatctccttatgttaatttaaagaaaaggttggttactac (SEQ ID NO: 30) |

### Resulting transformation vectors:

| | |
|---|---|
| plCF1320 | P60 translation control element |
| plCF1321 | SSP-7 translation control element |
| plCF1322 | YeO3-12 translation control element (see Figure 8) |
| plCF1323 | gh1 translation control element. |

### Generation of tobacco plastid transformants

Tobacco seeds (Nicotiana tabacum cv. Petite Havana) were surface sterilized (1 min in 70% ethanol, 10 min in 5% Dimanin C, Bayer, Leverkusen, Germany), washed 3 times for 10 min in sterile H₂O and put on SCN-medium (Dovzhenko *et al.,* 1998). Plants were grown at 25°C in a 16h light/8h dark cycle (0.5 - 1 W/m², Osram L85W/25 Universal-White fluorescent lamps). Protoplast isolation was made according to Dovzhenko *et al.* (1998). Transformation with vectors plCF1213, pICF1320, pICF1321, pICF1322, and pICF1323 using polyethyleneglycol (PEG) was performed as decribed in Koop *et al.* (1996), and alginate embedding according to Dovzhenko *et al.* (1998). After one week of culture in liquid medium, cells were transferred to agar-solidified RMOP-medium (Svab *et al.,* 1990) containing 25µg/ml kanamycin to select for transformants. Green regenerants were retrieved after 3-8 weeks of culture and were transferred to individual plates. Individual lines were subjected to repeated cycles of shoot generation by cutting small leaf pieces, which form new regenerates on RMOP-medium with 15 µg/ml kanamycin. Rooting of selected regenerants was performed on SCN-medium. Plastid transformation was confirmed by molecular analysis (PCR and Southern analysis according to standard methods).

### Analysis of transplastomic plants for GUS expression

### 1) Staining of leaves and T1 seedlings (Jefferson et al., 1987)

Activity of the GUS-protein can be visualized by staining of tissue material with X-Gluc (5-bromo-4-chloro-3-indolyl-β-D-glucuronide). X-Gluc is split by GUS-activity into glucuronide and 5-bromo-4-chloro-indole, which builds dimers in form of a blue indigo-derivate, which can be seen as blue staining of the tissue.

Leaf pieces of greenhouse plants or 2 week old T1-seedlings were incubated in X-Glucstaining solution (100 mM NaH₂PO₄/Na₂HPO₄, 1 mM EDTA, 1 mM potassium hexacyanoferrate (II), 1 mM Potassium hexacyanoferrate (III), 0.3% Triton-X-100, 1 mM X-Gluc; pH 7.0) at 37°C for 6-16 h. After staining, the tissue material was repeatedly treated with 70% ethanol to remove the chlorophyll. Blue staining of the tissue is a proof for presence and activity of the *uid*A-gene in the analyzed material. Although this method is not quantitative, some information can be obtained from the time period that is needed for staining of the tissue. In samples, where the GUS activity is high (i.e. high expression of the *uidA*-gene), staining of tissue and/or solution can be seen within some hours, whereas in samples, where only a weak GUS activity is present, no staining can be seen without removal of the chlorophyll. For leaves and seedlings of plants with control element YeO3-12 blue staining was visible after some hours, whereas leaves and seedlings of plants with control element P60 needed over night incubation in staining solution to develop a blue color. Regardless of the time needed for staining, transgenic lines for all four control elements were positive in GUS staining (wild-type and GUS-positive seedlings depicted in figure 4).

### 2) SDS-PAGE

100 mg of leaf tissue from 4 week old T1-plants grown from seed was homogenized in 200 µl GUS extraction buffer (50 mM NaH₂PO₄, 10 mM EDTA, 10 mM 2-mercaptoethanol, 1 mM PMSF, 0.1 % N-lauroylsarcosine, 0.1 % Triton X-100 pH 7.0) in a bead mill for analysis by SDS-polyacrylamide-gel-electrophoresis (SDS-PAGE). The suspension was centrifuged at 13000 rpm at 4°C for 1 hour and the supernatant (raw extract) was quantified by Bradford analysis for protein concentration, enzymatic assay and gel-electrophoresis. 100 µl of the clarified supernatant were mixed with 10 µl 20x DTT (2M) and 30 µl 5x loading dye (Fermentas) and heated to 100°C for 5 min. 10 µl of the denatured samples were loaded on a 10% SDS-PAGE-Gel (Serva) and run in a Hoefer SE260 electrophoresis apparatus. A sample from seedlings of a line with the T7 control element was included as a reference. Strong bands were visible for control element YeO3-12 (Figure 2, lane 4) and for control element T7 (Figure 2, lane 5), which indicated a very high expression of the *uidA*-gene unter the control of element YeO3-12 (Figure 2).

### 3) Enzymatic assay

5 µl raw extract or diluted raw extract was added to 200 µl assay buffer (50 mM NaH₂PO₄, 10 mM 2-mercaptoethanol, 1 mM p-nitrophenyl-glucuronide, 0.1 % Triton X-100 pH 7.0) in a microplate. The assay was mixed for 30 sec (level 2) and then absorbance at 415 nm was monitored in 1 min intervals for 60 min at 37 °C (Bio-Tek EL8081U-PC microplate reader). 25 ng GUS (type X-A, Sigma) was used as standard. The enzymatic activity was calculated from the slope of the linear segment of the absorbance/time graph. The percentage total soluble GUS-protein (% TSP) was calculated from the GUS-activity and the total protein in the sample as determined by Bradford-assay. Three samples each from two different plants of each line (construct) were assayed. The average value and standard deviation of each construct is shown in Figure 3.

### Example 2: Construction of new transient selection cassettes using translation control element YeO3-12

### Transient selection cassette aphA-6

The 16S-promoter from the tobacco plastome is amplified using oligos o473 5'-gcctaggcctgcaggctcccccgccgtcg-3' (SEQ ID NO: 31) and o474 5'-ggaagacccctcccagaaatatagcc-3' (SEQ ID NO: 32) from genomic DNA of tobacco. The resulting DNA-fragment is first restricted with PstI and treated with T4-polymerase to remove the overhang and subsequently cut with Bpil. The YeO3-12 translation control element is amplified using oligos o762 5'-ggagaagactgggagggagagaccatagatgactacaatgg-3' (SEQ ID NO: 33) and o765 5'-ggaccatggaaatgctagccatgttgaatctcc-3' (SEQ ID NO: 34) from plCF1322 and cut with enzymes BpiI and NcoI. Vector pICF1145 is restricted first with AscI and treated with T4-polymerase to produce a blunt end and afterwards cut with NcoI. Both fragments are ligated into pICF1145 to obtain vector pICF-P16SYeO-aphA-6 (see Figure 9A).

### Transient selection cassette aadA

Vector pICF-16SYeO-aphA-6 is cut with Ncol and Sphl and ligated with the aadA-fragment excised from pKCZ (Zou et al., 2003) with the enzymes Ncol and Sphl. The resulting vector pICF-P16SYeO-aadA contains a high expression selection cassette allowing for efficient selection of plant tissues with spectinomycin or streptomycin (see figure 9 B).

### Transient selection cassettes with antibiotic selection gene and visible marker GFP

The coding region of smGFP is amplified from pICF10501 (WO 2005/054481) using oligos oDB-GFP 5'-tggctagcatttccatggggatgagtaaagg-3' (SEQ ID NO: 35) and oGFP3'Pst 5'-ggactgcagttatttgtatagttcatccatgc-3' (SEQ ID NO: 36). The fragment is restricted with enzymes Nhel and Pstl and ligated together with the rps19 linker fragment Sdal/Ncol (see example 1; protruding ends of PstI and SdaI are compatible) into the transient selection cassette vector pICF-P16SYeO-aphA-6 cut with Nhel and Ncol. The same ligation is made with vector pICF-P16SYeO-aadA. The resulting new vectors are pICF-P16S-Ye-GFP-aphA-6 and pICF-P16S-Ye-GFP-aadA, respectively (see Figure 9 C and D).

Each of the four selection cassettes is excised as blunt end fragment by restriction with XhoI and NotI and subsequent T4-Polymerase treatment. The transient selection cassette is ligated into pICF1207, which is restricted with Sapl and treated with T4-Polymerase. The selection cassette can be inserted in both orientations.

Vectors with efficient transient selection cassettes are needed to generate marker-free transplastomic plants in a two-step process (WO03/004658).

### Example 3: Construction of a vector to produce a herbicide resistant plant without an antibiotic resistance marker gene

The bar gene, which detoxifies phosphinothricin, is amplified with oligos oDB-bar 5'-tggctagcatttccatgggcccagaacgacgcccg-3' (SEQ ID NO: 37) and obar-AscI 5'-atggcgcgcctcagatctcggtgacgggcaggaccg-3' (SEQ ID NO: 38) from plasmid pUM71 (lamtham and Day, 2000) to introduce an Nhel-site at the 5'-end and an Ascl-site at the 3'-end of the gene. The amplified fragment is restricted with NheI and AscI and ligated into pICF1207 cut with NheI and AscI. The resulting vector prbcL-bar is restricted with SapI and treated with T4-polymerase to insert one of the transient selection cassettes described in Example 2.

Insertion of cassette P16S-Ye-GFP-aphA-6 allows detection of early transformation events on kanamycin selection not only by visual screening for green calli but also by screening for GFP-fluorescence. Once the transformed lines are identified the selection is changed to phosphinothricin to obtain antibiotic resistance marker free transplastomic plants. Complete loss of the transient selection cassette is monitored by loss of GFP fluorescence in the tissue.

### Example 4: Construction of a high expression vector for the insertion site trnl-trnA for transformation of additional species

The combination of P16S-promoter and YeO3-12 control element is amplified from vector pICF-P16SYeO-aphA-6 (see Example 2; Figure 9 A) using oligos o473 5'-gcctaggcctgcaggctcccccgccgtcg-3' (SEQ ID NO: 31) and o765 5'-ggaccatggaaatgctagccatgttgaatctcc-3' (SEQ ID NO: 34) and restricted with the enzyme Ncol. The *uidA* (GUS)-fragment is excised from vector pICF1207 with enzymes NcoI and AscI. Vector pICF1145 is restricted first with Xhol, treated with T4-polymerase to produce blunt ends and afterwards restricted with AscI. The fragments P16S-YeO3-12 and uidA are ligated into vector pICF1145 to give vector pICF-P16S-Ye-GUS-aphA-6. The *trn*A-flank is excised from vector A0 (Mühlbauer *et al.,* 2002) with enzymes PvulI and KpnI. The complete expression cassette is excised from vector pICF-P16S-Ye-GUS-aphA-6, first restricted with enzyme SacI, treated with T4-polymerase and subsequently restricted with Bsp120I. Both fragments are ligated into the pBluescript KSII+ vector (Stratagene) cut with enzymes Bsp120I and KpnI. The resulting vector is plCF -P16S-Ye-GUS-aphA-6-trnA. The second flank is excised from vector A0 (Mühlbauer *et al.,* 2002) with enzymes Eagl and Pvull and ligated into vector pICF-P16S-Ye-GUS-aphA-6-trnA cut with Eagl and Aatl (restriction site in primer o473: aggcct) to give the finished vector pICF-trnl-P16S-Ye-GUS-aphA-6-trnA.

### Example 5: Expression in bacteria

Vector pICF-trnl-P16S-Ye-GUS-aphA-6-trnA is transformed into electrocompetent E. coli. Transformed colonies are selected on LB-medium supplemented with 150 mg/l ampicillin. Transformed cells are grown in LB-medium supplemented with 150 mg/l ampicillin to an optical density of 0.8 at 600 nm. Cells are collected by centrifugation and 5 g of the pellet is suspended in 10 ml GUS extraction buffer (50 mM NaH2PO4, 10 mM EDTA, 10 mM 2-Mercaptoethanol, 1 mM PMSF, 0.1 % N-Lauroylsarcosine, 0.1 % Triton X-100 pH 7.0). Cells are disrupted by ultrasonic pulsing (20 pulses, microtip level 4, Sonifier B-12, Branson SONIC Power Company, USA). Cell debris is removed by centrifugation (13000 rpm, 20 min., 4 °C) and the supernatant (raw extract) is analyzed by SDS-PAGE and enzymatic assay as described in example 1. E. coli contains low endogenous GUS activity, but endogenous GUS expression is too low to be visible on a Coomassie stained gel. Visible GUS signals therefore indicate expression mediated by vector pICF-trnl-P16S-Ye-GUS-aphA-6-trnA. The enzymatic assay also can clearly discriminate between the low endogenous background activity in untransformed E. coli cells and high vector dependent expression in transformed E. coli cells.

### Example 6 : Plastid transformation of and expression in potato (Solanum tuberosum)

### Preparation of potato protoplasts and culture in thin-alginate-layers (TAL)

Plants of *S. tuberosum* cv. Walli are grown *in vitro* as sterile shoot cultures (20±1°C, 16h day, light intensity 75 ± 10 µmoles/m²/sec). New cultures are initiated every 2 months by transferring shoot tips (approx. 2 cm in length) to MS medium (Murashige and Skoog, 1962) in glass tubes (2.5 x 20 cm). Young fully expanded leaves (selected from 3-4 week old plants) are used routinely for protoplast isolation. Leaves are cut into 1 mm stripes with a scalpel and pre-plasmolysed in 10 ml of MMM-550 medium. MMM-550 medium contains 4.066 g/l MgCl₂6H₂0, 1.952 g/l 2-(N-morpholino) ethanesulfonic acid (MES) and ~86 g/l mannitol (adjusted to 550 mOsm and pH 5.8). After 1 hour of incubation in the dark the MMM-550 is removed and replaced with 10 ml of MMS-550 medium containing 0.4% w/v Macerozyme R10 and 0.4% Cellulase R10. MMS-550 medium contains 4.066 g/l MgCl₂6H₂0, 1.952 g/I MES and -150 g/l sucrose (adjusted to 550 mOsm and pH 5.8). The leaf explants in enzyme solution are incubated for 16 hours in the dark at 25°C without shaking. The following day the digestion is filtered through a 100 µm sieve into a centrifuge tube and carefully overlaid with 2 ml of MMM-550 medium and centrifuged (10 min, 70 xg). Intact protoplasts are collected from the band at the interface and washed once by resuspending in 10 ml of potato protoplast culture medium followed by centrifugation (10 min, 50 xg). The protoplast culture medium contains 133.75 mg/l NH₄Cl, 950 mg/l KNO₃, 220 mg/l CaCl₂·2 H₂O, 185 mg/l MgSO₄7H₂O, 85 mg/l KH₂PO₄, B5 microelements (Gamborg et al. 1968), MS Fe-EDTA (Murashige and Skoog, 1962), 100 mg/l myo-inositol, 100 mg/l glutamine, 100 mg/l casein hydrolysate, 1 mg/l nicotinic acid, 10 mg/l thiamine hydrochloride, 1 mg/l pyridoxine hydrochloride, 250 mg/l xylose, 975 mg/l MES, 2 mg/l naphthalene acetic acid (NAA), 0.2 mg/l 2,4-dichlorophenoxyacetic acid (2,4-D), 0.5 mg/l 6-benzylaminopurine (BAP) and -94 g/I glucose (adjusted to 550 mOsm and pH 5.8). Protoplasts are counted and resuspended at 2x the required final plating density in protoplast culture medium (2.0 x 10⁵/ml) and mixed with an equal volume of 1.2% w/v alginic acid prepared in MMM-550 medium. Thin alginate layer culture in polypropylene grids is as described in Dovzhenko et al. (1998). Following solidification of the alginate matrix, grids are transferred to 5cm Petri dishes containing 2 ml of protoplast culture medium. Protoplasts are incubated for one day in the dark (26±1°C) and then transferred to standard culture room conditions for further development (26±1°C, 16h day, light intensity 75 ± 10 µmoles/m²/sec). Twelve to 15 days after protoplast embedding the grids containing microcolonies (approx. 8 cells) are transferred to 9 cm dishes containing 25 ml of SH-1 medium solidified with 0.7% w/v agar. SH-1 medium contains 267.5 mg/l NH₄Cl, 1900 mg/l KNO₃, 440 mg/l CaCl₂2H₂O, 370 mg/l MgSO₄7H₂O, 170 mg/l KH₂PO₄, MS microelements and Fe-EDTA (Murashige and Skoog, 1962), Nitsch vitamins (Nitsch and Nitsch, 1969), 40mg/l adenine sulphate, 100 mg/l casein hydrolysate, 975 mg/l MES, 0.1 mg/I NAA, 0.5 mg/l BAP, 10 g/I sucrose and 50 g/I mannitol (adjusted to pH 5.8).

### Transformation of protoplast-derived microcolonies by bombardment

Two days after plating on solid medium, protoplast-derived colonies are bombarded with aliquots of gold loaded with 25 µg of vector pICF-trnl-P16S-Ye-GUS-aphA-6-trnA using the particle coating and bombardment conditions described in Mühlbauer et al. (2002). Selection of transformants is based on the resistance to the antibiotic kanamycin, conferred by the *aphA-6* gene product. One day after bombardment grids are transferred to dishes containing agar-solidified SH-1 medium + 25 mg/l kanamycin and subcultured every 3 weeks to fresh selection dishes.

### Selection and regeneration of transplastomic potato shoots

Plastid transformants are observed as small green microcolonies after 8-12 weeks of selection (non-transformed tissues are bleached on SH-1 medium containing kanamycin). Individual colonies (approx. 1 mm in diameter) are transferred to 5 cm dishes containing SH-1 medium + 25 mg/l kanamycin for enlargement. For regeneration, calli (approximately 5 mm in diameter) are transferred to SH-2 medium solidified with 0.7% w/v agar containing 10 mg/l kanamycin. SH-2 medium is identical to SH-1 medium (see above) except that the NAA is replaced with 0.1 mg/l indole-3-acetic acid (IAA), BAP is replaced with 1 mg/l zeatin and the mannitol content is reduced from 50 g/I to 36 g/I. Shoots can be removed from regenerating calli after 6-8 weeks of culture on SH-2 medium and transferred to antibiotic-free MS medium for rooting and further development.
Confirmation of transformation can be made using GUS histochemical straining as described in Example 1.

### ANNEX:

SEQ ID NO: 1 (translation control element from φYeO3-12)
   5'- gagagaccatagatgactacaatggttgaatcacctgagcacagaactttgtggtcactcccgtaggtgaaacat tgagaaccaactcgattcaagtagtaaccaaccttttctttaaattaacataaggagattcaac-3'
SEQ ID NO: 2 (truncated translation control element from φYeO3-12)
   5'-acattgagaaccaactcgattcaagtagtaaccaaccttttctttaaattaacataaggagattcaac-3'
SEQ ID NO: 3 (modified translation control element)
   5'-caattgagacccaacgctaacaaagtagtaaccaaccttttctttaaatggatccaaggagattcaat-3'

### REFERENCES

Buhot, L.; Horvàth, E. M.; Medgyesy, P.; Lerbs-Mache, S. The Plant Journal 2006, 46, 700-7.
Bateman, J.M.; Purton, S. Molecular and General Genetics 2000, 263, 404-10.
Chen Z.; Schneider T.D. Nucleic Acids Research 2005, 33, 6172-87.
Dovzhenko, A.; Bergen, U.; Koop, H.-U. Protoplasma 1998, 204,114-118.
Eibl C., Zou Z., Beck A., Kim M., Mullet J., Koop H.U. The Plant Journal 1999, 19, 333-345. Galvin S. B., 1998, Curr. Opin. Biotechnol., 9, 227-232*.*
Gamborg, O.L.; Miller, R.A.; Ojima, K. Exp Cell Res 1968, 50, 151-158.
Golds, T. J.; Maliga, P.; Koop, H.-U. Bio/Technology 1993, 11, 95-97.
Gray M. W., Origin and Evolution of Plastid Genomes and Genes, in: Bogorad L. and Vasil I. K. (eds.), Cell Culture and Somatic Cell Genetics of Plants, Volume 7A, Academic Press, San Diego, 1991.
Herz, S.; Fussl, M.; Steiger, S.; Koop, H.-U. Transgenic Research 2005, 14, 969-982. lamtham, S.; Day, A. Nature Biotechnology 2000, 18, 1172-6.
Jefferson, R. A.; Kavanagh, T. A.; Bevan, M.W. The EMBO Journal 1987, 6, 3901-7.
Klaus, S. M. J.; Huang, F.-C.; Golds, T. J.; Koop, H.-U. Nature Biotechnology 2004, 22, 225-229.
Koop, H.-U.; Steinmüller, K.; Wagner, H.; Rossler, C.; Eibl, C.; Sacher, L. Planta 1996, 199,193-201.
Koop, H.-U.; Herz, S.; Golds, T.J.; Nickelsen, J., Trends in Current Genetics 2007 Kuroda, H.; Maliga, P. Plant Physiology 2001, 125, 430-6*.*
Kuroda, H.; Maliga, P. Nucleic Acids Research 2001, 29, 970-5.
Kuroda, H.; Maliga, P. Plant Physiology 2002, 129, 1600-6.
Marechal-Drouard L., Kuntz M., Weil J. H., tRNAs and tRNA Genes of Plastids, in: Bogorad L. and Vasil I. K. (eds.), Cell Culture and Somatic Cell Genetics of Plants, Volume 7A, Academic Press, San Diego, 1991.
Maliga, P. Annual Review of Plant Biology 2004, 55, 289-313.
Maliga, P. Photochem. Photobiol. Sci., 2005, 4, 971-976.
McBride, K. E.; Schaaf, D. J.; Daley, M.; Stalker, D. M. Proceedings of the National Academy of Sciences of the United States of America 1994, 91, 7301-5.
Mühlbauer, S. K.; Lössl, A.; Tzekova, L.; Zou, Z.; Koop, H.-U. The Plant Journal 2002, 32, 175-84.
Murashige, T. ; Skoog, F. Physiol Plant 1962, 15, 473-497.
Newell, C. A.; Birch-Machin, I.; Hibberd, J. M.; Gray, J. C. Transgenic Research 2003, 12, 631-4.
Nitsch, J.P.; Nitsch, C. Science 1969, 169, 85.
Palmer J. D., Plastid Chromosomes: Structure and Evolution, in: Bogorad L. and Vasil I. K.(eds.), Cell Culture and Somatic Cell Genetics of Plants, Volume 7A, Academic Press, San Diego, 1991*.*
Reddy, V. S.; Leelavathi, S.; Selvapandiyan, A.; Raman, R.; Giovanni, F.; Shukla, V.; Bhatnagar, R. K. Molecular Breeding 2002, 9, 259-269.
Rogalski, M.; Ruf, S.; Bock, R. Nucleic Acids Research 2006, 34, 4537-45.
Sriraman, P.; Silhavy, D.; Maliga, P. Nucleic Acids Research 1998, 26, 4874-4879.
Staub, J. M.; Maliga, P. The EMBO Journal 1993, 12, 601-6.
Staub, J. M.; Maliga, P. The Plant Journal 1995, 7, 845-8.
Staub, J. M.; Garcia, B.; Graves, J.; Hajdukiewicz, P. T. J.; Hunter, P.; Nehra, N. S.; Paradkar, V.; Schlittler, M.; Carroll, J. A.; Spatola, L.; Ward, D.; Ye, G.-N.; Russell, D. A. Nature Biotechnology 2000, 18, 333-8.
Stern D.B., Gruissem W.,1987, Cell, 24,1145-1157.
Stern, D. B.; Higgs, D. C.; Yang, J. Trends in Plant Science 1997, 2, 308-315.
Sullivan, M. B.; Coleman, M. L.; Weigele, P.; Rohwer, F.; Chisholm, S. W. PLoS Biol 2005, 3, e144.
Svab, Z.; Hajdukiewicz, P. T. J.; Maliga, P., Proceedings of the National Academy of Sciences of the United States of America 1990, 87, 8526-8530.
Svab, Z.; Maliga, P. Proceedings of the National Academy of Sciences of the United States of America 1993, 90, 913-7.
Zhou, Y.-X.; Lee, M. Y.-T.; Ng, J. M.-H.; Chye, M.-L.; Yip, W.-K.; Zee, S.-Y.; Lam, E. World Journal of Gastroenterology 2006, 12, 306-12.
Zoubenko, O. V.; Allison, L. A.; Svab, Z.; Maliga, P. Nucleic Acids Research 1994, 22, 3819-24.
Zou, Z; Eibl, C; Koop, H.-U., Molecular Genetics and Genomics, 2003, 269, 340-9.

## Claims

1. Nucleic acid comprising a translation control element and, downstream thereof, a non-yersinia phage coding sequence to be expressed in plant plastids or bacteria, said translation control element comprising
(i) a sequence of SEQ ID NO: 1;
(ii) a sequence of SEQ ID NO: 2; or
(iii) a sequence having a sequence identity of at least 75 % to the sequence of SEQ ID NO: 2;
or a complementary nucleic acid having a sequence complementary to that of said nucleic acid.

2. The nucleic acid according to claim 1, wherein said sequence of item (iii) has a sequence identity of at least 85 %, preferably of at least 90 %, to the sequence of SEQ ID NO:2.

3. The nucleic acid according to claim 1, wherein said sequence of item (iii) comprises a ribosome binding site upstream of a start codon of said coding sequence to be expressed.

4. The nucleic acid according to claim 3, wherein said ribosome binding site comprises nucleotide sequence AGGAG.

5. The nucleic acid according to any one of claims 1 to 4, further comprising a transcription promoter active in plant plastids upstream of said translation control element.

6. The nucleic acid according to any one of claims 1 to 5, further comprising a second coding sequence to be expressed and upstream thereof a second translation control element.

7. The nucleic acid according to claim 6, wherein said second translation control element is a bacteriophage T7G10 translation control element.

8. A vector for plastid transformation of a plant, plant tissue or plant cells, said vector comprising said nucleic acid or said complementary nucleic acid as defined in any one of claims 1 to 7.

9. The vector according to claim 8, further comprising homologous flanks flanking said nucleic acid for plastome integration of said nucleic acid by homologous recombination.

10. A plant, plant tissue or plant cells comprising in plastids thereof a nucleic acid comprising a translation control element and, downstream thereof, a coding sequence to be expressed in plastids of said plant, plant tissue or plant cells, said translation control element comprising
(i) a sequence of SEQ ID NO: 1; or
(ii) a sequence of SEQ ID NO: 2; or
(iii) a sequence having a sequence identity of at least 75 % to the sequence of SEQ ID NO: 2;
or a complementary nucleic acid having a sequence complementary to that of said nucleic acid.

11. Said plant, plant tissue or plant cells according to claim 10, comprising said nucleic acid in plastomes of said plant, plant tissue or plant cells.

12. Said plant, plant tissue or plant cells according to claim 10 or 11, further comprising a second translation control element and, downstream thereof, a second coding sequence to be expressed in plastids of said plant, plant tissue or plant cells, whereby said translation control element defined in claim 10 and said second translation control element are different translation control elements.

13. Said plant, plant tissue or plant cells according to any one of claims 10 to 12, wherein said plant is a dicotyledonous or a monocotyledonous plant, or said plant tissue or plant cells are derived from a dicotyledonous or a monocotyledonous plant.

14. A process of expressing a coding sequence of interest in plastids of a plant, plant tissue or plant cells, said process comprising providing a plant, plant tissue or plant cells with a nucleic acid as defined in claim 10 or with the vector of claim 8.

15. A process of producing a transplastomic plant, plant tissue or plant cells, said process comprising providing a plant, plant tissue or plant cells with a nucleic acid as defined in any one of claims 1 to 7 or the vector of claim 8, and selecting a plant, plant tissue or plant cells transformed on their plastome.

16. A bacterial cell comprising a nucleic acid comprising a translation control element and, downstream thereof, a non-yersinia phage coding sequence to be expressed in said bacterial cell, said translation control element comprising
(i) a sequence of SEQ ID NO: 1; or
(ii) a sequence of SEQ ID NO: 2; or
(iii) a sequence having a sequence identity of at least 75 % to the sequence of SEQ ID NO: 2; or
or a complementary nucleic acid having a sequence complementary to that of said nucleic acid.

17. A process of expressing a coding sequence of interest in a bacterium, comprising introducing a nucleic acid as defined in any one of claims 1 to 7 or the vector of claim 8 into said bacterium.

18. Use of a nucleic acid comprising a nucleic acid sequence selected from the group consisting of SEQ ID NO: 1, SEQ ID NO: 2, SEQ ID NO: 3, and a sequence having a sequence identity of at least 75 % to the sequence of SEQ ID NO: 1 or 2 for expressing a sequence of interest in a plant, in plant tissue, in a plant cell, or in bacteria.
